# EUROPEAN PATENT APPLICATION

(11) **EP 3 832 659 A1**
(43) Date of publication of application: **09.06.2021**
(21) Application number: 19212851.0
(22) Date of filing: 02.12.2019
(51) Int. Cl.: G16H 20/40, A61B 34/10

(54) **APPARATUS AND METHOD FOR ASSISTING PRECISION ABLATION AND THE PLANNING THEREOF**

(71) Applicant: Koninklijke Philips N.V., 5656 AG Eindhoven (NL)
(72) Inventor: HONG, Adeline, 5656 AE Eindhoven (NL); YIN, Tinghui, 5656 AE Eindhoven (NL); CHEN, Zhouye, 5656 AE Eindhoven (NL); GUO, Wei, 5656 AE Eindhoven (NL); LI, Kai, 5656 AE Eindhoven (NL); BAI, Xiang Hui, 5656 AE Eindhoven (NL); XU, Erjiao, 5656 AE Eindhoven (NL); ZHENG, Rongqin, 5656 AE Eindhoven (NL)
(74) Representative: Philips Intellectual Property & Standards

(57) **Abstract**

The present invention proposes an apparatus and a method for assisting an ablation procedure on a target region of a subject. The apparatus comprises a user interface, a risk estimator, a risk alert positioning unit. The user interface is configured to visualize an image of the target region and at least one critical region of the subject. The risk estimator is configured to estimate an impact of the ablation on each of the at least one critical region, and to generate risk alert information based on the estimated impacts and a risk alert rule. The risk alert information includes one or more risk alert, each of which includes information indicative of the corresponding critical region. The risk alert positioning unit is configured to obtain alert position information of each of the at least one critical region. The alert position information of a critical region is indicative of an alert position for the critical region in the image where the risk alert is to be visualized, and the alert position is at or adjacent to a position of the critical region in the image. The risk estimator is further configured to, in response to any change of the estimated impact or the risk alert rule, automatically update the risk alert information; and the user interface is further configured to, in response to any change of the risk alert information, automatically visualizing, in the image, the one or more risk alerts based on the risk alert information and the alert positioning information. The proposed apparatus provides an intuitional and interactive visualization of the risk relating to injuring tissue/organ other than the ablation target. For example, the risk alert(s) can be vividly visualized with respect to the corresponding critical region, which is especially beneficial when there are more than one critical region in the image. Moreover, the risk alert(s) are automatically updated in response to any change of the impact, including user-initiated change, which enable a more efficient workflow either for ablation planning or for a real ablation procedure.

## Description

### FIELD OF THE INVENTION

The present invention relates to an apparatus and method for assisting clinical procedure such as precision ablation and the planning thereof, and more particularly to an apparatus and method for assisting an ablation procedure.

### BACKGROUND OF THE INVENTION

Common treatment options for liver cancer patients include surgical resection, liver transplant, chemotherapy, trans-arterial chemoembolization (TACE), percutaneous ethanol injection (PEI), cryotherapy, radiofrequency ablation (RFA), microwave ablation (MWA), high-intensity ultrasound (HIFU), and newer ablative modalities such as irreversible electroporation (IE). RFA and MWA, as minimally invasive therapies, have developed rapidly in the past 20 years and are among the most important methods for liver cancer treatment. In RFA, MWA, HIFU, IE and other kinds of ablation procedures, most risks are associated with injury of surrounding tissue, most commonly thermal damage when the ablation causes temperature increase in neighboring organs, which happens in 2% of the cases.

Conventionally and still very popularly, clinicians acquire 2D ultrasound images from different angles while scanning the patient's liver to judge the relative distance between the critical organs to the ablation target. The lack of accurate measures can lead to severe negative outcomes. When the distance is underestimated, and no preventative measures are taken, surrounding tissues can be damaged and putting the patient's health and life in danger. A slightly advanced solution that has been practiced in the clinics is to plan the ablation using reconstructed 3D MR/ CT or use 3D ultrasound right before the procedure to assess the patient's anatomical arrangements and positions. However, this requires the clinicians to rotate the 3D volume to check every possible angle that the critical organs may touch the lesion/ablation target. Clinicians can easily miss a threatening location using manual rotation and evaluation.

### SUMMARY OF THE INVENTION

Therefore, it would be advantageous to provide an improved technical solution for assisting an ablation procedure, such as assisting clinicians to plan, prepare for, and/or perform the ablation procedure.

In accordance with an embodiment of a first aspect of the present invention, there is provided an apparatus for assisting an ablation procedure on a target region of a subject. The apparatus comprises a user interface, a risk estimator, and a risk alert positioning unit. The user interface is configured to visualize an image of the target region and at least one critical region of the subject. The risk estimator is configured to estimate an impact of the ablation procedure on each of the at least one critical region, and to generate risk alert information based on the estimated impacts and a risk alert rule. The risk alert information includes one or more risk alert, each of which includes information indicative of the corresponding critical region. The risk alert positioning unit is configured to obtain alert position information of each of the at least one critical region. The alert position information of a critical region is indicative an alert position of the critical region in the image at which the risk alert is to be visualized. The alert position is at or adjacent to a position of the critical region in the image. The risk estimator is further configured to, in response to any change of the estimated impact, automatically update the risk alert information. The user interface is further configured to, in response to any change of the risk alert information, automatically visualizing, in the image, the one or more risk alerts based on the risk alert information and the alert positioning information.

In this way, whenever there is a change of the estimated impact (e.g. caused by a change of the position of the ablation device, a change of the energy or duration of the ablation, or a change of the number of ablation instances etc.) or a change of the risk alert rule (e.g. caused by a user input), the risk alert can not only be immediately updated and visualized in response to such change without additional manual operations, but also be visualized in the same image which illustrates the target region and the critical regions and the position where the risk alert is visualized indicates which critical region the alert is associated with. All such visualizations can improve the overall procedure workflow and efficiency. All such visualizations can provide clinicians with easy-to-read quality-measure and/or quantitative-measures and assist the clinician to make confident judgment to improve the overall procedure workflow and efficiency.

The target region refers to a region to be ablated in an ablation procedure. Sometime, there is a difference between a tissue structure (often called an ablation target, such as a tumor) which the ablation procedure aims to ablate and a region (often called an ablation region) which the ablation procedure actually ablates or would ablate according to the ablation plan, and it is well-known that the ablation region shall sufficiently cover the ablation target within certain residual tolerance. However, in the context of this patent application, there is no intention to differentiate "ablation target" and "ablation region" because the difference is irrelevant for the invention, and the term "target region" can refer to either of them.

A critical region refers to any region where tissue and/or organ therein could be injured during the ablation procedure and such injury is desirably avoided. Typically, the critical region is a region surrounding the target region and possibly influenced by the ablation, such as bladder, gall bladder, intestine, vessel drainage system, etc. The critical region(s) can be predefined for certain ablation procedure, and/or user-defined.

The impact of the ablation can be any suitable parameter indicative an impact on a critical region caused by the ablation procedure. For example, the estimated impact can be the distance between the target region and the critical region, because smaller distance typically suggests stronger impacts.

The risk alert rule can any rule defining when or under which conditions to generate a risk alert. For example, a risk alert rule can be to generate a risk an alert for a critical region when the distance between the target region and the critical region is smaller a threshold value. In some other examples, the risk alert rule can be more complex or sophisticated as will be further described here below.

The image of the target region and at least one critical region refers to an image which is capable of reflecting the relative size and/or position of the target region and the at least one critical region. The image of the target region and at least one critical region can be a 2D or a 3D image, can be a medical image or any image synthesized from medical images, and can also be a VR (virtual reality) or AR (augmented reality) image. The user interface can be a display, a projector, a pair of glasses or any type of device suitable for visualizing an image to a human being. In some examples, the image includes respective indications for the target region and/or the at least one critical region so as to assist users to identify them.

The alert position for a critical region is defined as a position in the image where the risk alert is to be visualized and is at or adjacent to the position of the critical region. Here, the persons skilled in the art should readily appreciate that the expression "adjacent to a critical region" refers to a position that is closer to the critical region than any other critical region such that no one would misread the risk alert as being associated with any other critical region.

The alert information includes one or more risk alert, and each risk alert is associated with a critical region. In some examples, risk alerts can be classified into a number of risk levels according to the severity of the risk, and each risk alert can be further associated with an alert level. Different level of risk alerts can be visualized in different ways, such as via different colors. The visualization of risk alert can be implemented in various ways. In some example, the associated critical region can be highlighted in any suitable way so as to indicate a risk associated thereto. Additionally and alternatively, a predefined icon or similar can be visualized at the alert position of the associated critical region.

In some embodiments, the risk estimator is further configured to obtain spatial relationship between the target region and the at least one critical region, to obtain risk reference data relating to the spatial relationship for each of the at least one critical region, and to estimate the impact of the ablation on each of the at least critical region based on the spatial relationship, the risk reference data and the risk alert rule. For example, the spatial relationship between a target region and a critical region can be any suitable parameter indicative the distance between the target region and the critical region, such as a center-to-center distance, an edge-to-edge distance or the like. The risk reference data can be for example certain threshold value. The risk reference data for one critical region can be different from that for the other critical region.

In same examples, the risk reference data includes a distance threshold for relative distance to the target region, and the user interface is further configured to visualize the distance threshold in the image. In an example, the distance threshold can be visualized via an isometric curve. In another example, the distance threshold can be visualized by indicating a region within which the relative distance to the target region is smaller than the distance threshold. Thus, it would further assist the clinicians to assess how far the critical region shall be kept away from the target region.

In some embodiments, the risk estimator is configured to obtain thermal distribution of the ablation over the at least one critical region, to obtain risk reference data relating to temperature for each of the at least one critical region, and to estimate the impact of the ablation on each of the at least critical region based on the thermal distribution, the risk reference data and the risk alert rule.

Ablations can be performed by either heating the target region or freezing the target region, and the surrounding tissue/structure could be injured due to heat conduction from the target region to the surroundings. Such heat conduction is typically not linear due to the different type of the surrounding tissues and/or the existence of heat sink such as vessels. Consequently, thermal distribution caused by the ablation can be non-uniform as well as vary over individual ablation procedures. Risk reference data relating to temperature for a critical region can be, for example, certain temperature upper limit or lower limit which indicates the tolerable temperature of the critical region without being injured. By obtaining thermal distribution, the risk estimator is able to more accurately estimate the impacts on the critical regions.

In an embodiment, the user interface can be configured to visualize the obtained temperature distribution. The temperature distribution or the thermal field resulting from the ablation is affected by many factors, and is not easy for the clinician to imagine or predict. The explicit visualization of such temperature distribution would enable an easy and direct way of accessing of such information.

In a further embodiment, the risk reference data includes a temperature threshold, and the user interface is further configured to visualize the temperature threshold based on the temperature threshold and the temperature distribution. In an example, the temperature threshold can be visualized via an isotherm in the image delineating the points where the temperature is equal to the threshold according to the temperature distribution. In another example, the distance threshold can be visualized by indicating a region within which the temperature exceeds the temperature threshold. The term "exceed the temperature threshold" means to be higher than the temperature threshold in case of a temperature upper limit or lower than the temperature threshold in case of temperature lower limit.

In some embodiments, the risk estimator is configured to obtain risk reference data for each of the at least one critical region based on at least one of anatomical type of the critical region, subject-specific condition of the critical region, type of the ablation procedure, and user input relating to risk reference data. In an example, the risk estimator can be configured to determine the type of the critical region and/or the type of ablation procedure, and to access certain predetermined look-up table to obtain the corresponding risk reference data for that type of critical region and/or that type of ablation procedure. In another example, the risk estimator can be configured to determine the risk reference data based on user inputs.

In this way, the risk reference data such as threshold values can be set different for different type of critical regions, for different type of ablation procedures, and/or for different user preference. For example, there are two critical regions each relating to a different organ/tissue type, and the threshold value for one critical region can be set as being larger than that of the other, because it is more sensitive or more vulnerable to the ablation process. Consequently, it allows a more flexible configuration for the risk alerts and can meet the various needs of the ablation procedures to provide better assistance.

In some embodiments, the risk estimator is further configured to obtain a position change of a critical region relative to the target region, to estimate the impact of the ablation on the critical region based on the position change of the critical region. Prior to the inventors of the present patent application, no one proposes to consider the position change of a critical region relative to the target region when assessing the impact of the ablation, because it is commonly understood that tissues or organs won't change positions expect for some periodic movement caused by cardiac or respiration motion. The inventors of the present patent application have recognized that the position of a critical region can be changed or to be changed by an external force or factor, and such position change would certainly change the impact of the ablation on the critical region. Sometimes, clinicians may do an open surgery to further separate the critical organs in danger away from the ablation target. Sometimes, clinician may use other different invasive or non-invasive ways to achieve such separation. For instance, clinicians may perform hydrodisection, during which saline is injected in the patient's abdomen to further separate the liver from the surrounding organs. The proposed risk estimator is able to estimate the corresponding impact on the critical region when the clinicians plan to perform or have performed such separation. In this way, it can provide real-time assistance for the clinician to decide whether the critical region needs to be further separated or not.

In some embodiments, the risk estimator can receive a user input indicating the position change of the critical region, which can occur during the planning of the ablation procedure or during the performance of the ablation procedure. In an embodiment, the risk estimator is further configured to track the position of the critical organ. For example, the risk estimator is further configured to receive a further image of the subject, and to monitor the position change of the critical region by analyzing the further image. The further image can be a medical image acquired by various modalities, such as X-ray, ultrasound, or visible light camera etc. Some other suitable tracking means, such as a tag, can also be applied. In another embodiment, the risk estimator is further configured to determine the position change of the critical region based on a user input relating to the position of the critical region. Any user input can be input via various ways, including but not limited to keyboards, joystick, touch screen, gesture, voice, etc.

In some embodiments, the risk estimator is further configured to obtain a position of an ablation device, and to estimate the impact of the ablation on each of the at least critical region further based on the obtained position of an ablation device. The position of the ablation device can be determined from a user input, can be received from a planning tool the ablation procedure during the planning process, or can be obtained by tracking the position of the ablation device during the ablation procedure.

In accordance with an embodiment of a second aspect of the present invention, there is proposed a method of assisting an ablation procedure on a target region of a subject. The method comprises the steps of visualizing, via a user interface, an image of the target region and at least one critical region of the subject, estimating an impact of the ablation procedure on each of the at least one critical region, and generating risk alert information based on the estimated impacts and a risk alert rule. The risk alert information including one or more risk alert, each of which including information indicative of the corresponding critical region. The proposed method further comprises a step of obtaining alert position information of each of the at least one critical region, wherein the alert position information of a critical region is indicative an alert position for the critical region in the image where the risk alert is to be visualized, and the alert position being at or adjacent to a position of the critical region in the image. The method further comprises automatically update, in response to any change of the estimated impact or the risk alert rule, the risk alert information; and automatically visualizing, in response to any change of the risk alert information, in the image, the one or more risk alerts based on the risk alert information and the alert positioning information.

In accordance with an embodiment of a third aspect of the present invention, there is proposed a computer-readable medium comprising executable instructions, which when executed, cause a processor to perform the proposed method.

Other objects and advantages of the present invention will become more apparent and can be easily understood with reference to the description made in combination with the accompanying drawings.

### SHORT DESCRIPTION OF THE DRAWINGS

The present invention will be described and explained hereinafter in more detail in combination with embodiments and with reference to the drawings, wherein:
Fig.1 schematically illustrates an apparatus for assisting an ablation procedure on a target region of a subject in accordance with some embodiments of the present invention;
Fig.2 schematically illustrates a method of assisting an ablation procedure on a target region of a subject in accordance with some embodiments of the present invention; and
Fig.3A schematically illustrates an exemplary view visualized by a user interface in accordance with an embodiment of the present invention; and
Fig.3B schematically illustrates an exemplary view visualized by a user interface in accordance with an embodiment of the present invention.

The same reference signs in the figures indicate similar or corresponding features and/or functionalities.

### DETAILED DESCRIPTION OF THE EMBODIMENTS

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn to scale for illustrative purposes.

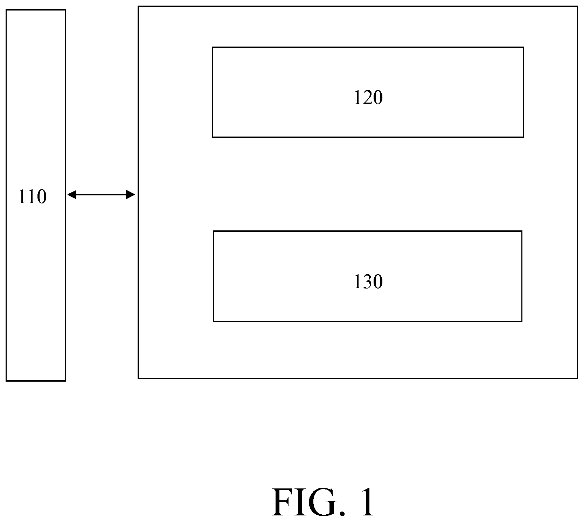

Fig.1 schematically illustrates an apparatus 100 for assisting an ablation procedure on a target region of a subject in accordance with some embodiments of the present invention. A target region refers to a region to be ablated in an ablation procedure. The target region can be, for example, a tumor or nodule in an organ (such as a liver) of the subject. A critical region refers to any region where tissue and/or organ therein could be injured during the ablation procedure and such injury is desirably avoided. For example, the at least one critical region in a liver ablation procedure includes one or more of gall bladder, intestine, vessel drainage system.

Referring to Fig. 1, the apparatus 100 comprises a user interface 110, a risk estimator 120 and a risk alert positioning unit. The user interface 110 are communicatively connected to both risk estimator 120 and the risk alert positioning unit.

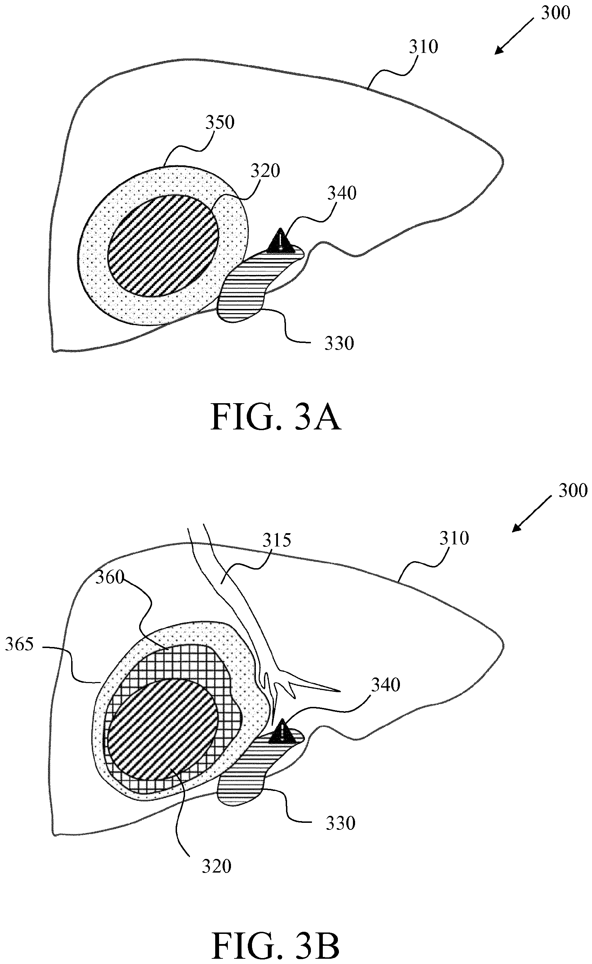

The user interface 110 is configured to visualize an image of the target region and at least one critical region of the subject. Each of Fig.3A and Fig.3B illustrates an exemplary image 300, wherein the target region 320 is part of an organ 310 (such as a liver). The image 300 is a two-dimensional view, but it is only for illustrative purpose. The image 300 include one critical region 330, but it can include two or more critical regions 330. The image 300 can be any kind of image suitable for visualizing the target region 320 and the at least one critical region 330 and their relative positions. In some embodiments, the image 300 can further include indicators (not illustrated) for the target region 320 and/or the at least one critical region 330, such as delineation of these regions and/or labels. The user interface 110 can receive image data relating to the image 300 from any data source, including a medical image acquisition apparatus such as an ultrasound device, or an image data repository such as PACS (picture archiving and communication system) or other type of healthcare informatics system.

The risk estimator 120 is configured to estimate an impact of the ablation procedure on each of the at least one critical region 330, and to generate risk alert information based on the estimated impacts and a risk alert rule.

Typically, the closer the critical region 330 to the target region 320 is located, on the critical region 330 will be more affected by the ablation of the target region 320, and the chance of the critical region being injured is higher. In an embodiment, the risk estimator 120 can estimate the impact on a critical region 330 by calculating the distance between the target region 320 and the critical region 330. The calculated distance can be defined as the distance between the center of the target region 320 and the center of the critical region. Alternatively, the calculated distance can be defined as the shortest distance between the center of the target region 320 and the boundary of the critical region 330. Alternatively, the calculated distance can be defined as the shortest distance between the boundary of the target region 320 and the boundary of the target region 330. The risk alert rule can be to generate a risk alert if the calculated distance is below a threshold value. In an embodiment, the user interface 110 can be configured to visualize such threshold value. In the example illustrated in Fig.3A, the target 320 is ovarian-shaped, and the boundary curve 350 is an isometric curve composed by any point whose distance to the boundary the center of the target region is equal to the threshold value. Additionally or alternatively, the user interface 110 can not only visualize the boundary curve 350 but also visualize the zone (filled by dots) between the boundary of the target region 320 and the boundary curve 350, often called risk zone.

The risk estimator 120 can obtain the spatial relationship between the target region 320 and the critical region 330 in various way. In an embodiment, the risk estimator 12 can determine the spatial relationship, such as the relative distance, by analyzing a medical image. In some embodiments, the risk estimator 120 can perform image segmentation to identify the target region 320 and the critical region 330. In some embodiments, the risk estimator 120 can detect the at least critical region 330 with or without user input. In an example, there can be a predetermined list of all critical regions (specific tissues, vessels or organs), and the risk estimator 120 can be configured to search the whole medical image to identify any critical region present in the whole medical image. In another example, the user can select a critical region via the user interface 110. The user can place one or more seed points on the critical region and the risk estimator 120 can then identify the whole critical region based on these seed points, or the user can delineate the whole critical region.

In some examples, the risk alert rule can be configured to generate more than one types of risk alerts, each indicating a different level of risk alerts. For example, a first type of risk alert if the calculated distance is smaller than a first threshold value and to generate a second type of risk alert (indicative a less severe alert) if the calculated distance is larger than the first threshold value but smaller than a second threshold value (larger than the first threshold value). Different types of risk alerts can be visualized in different ways, such as via different coloring scheme, or via different risk alert icons.

In some examples, the distance threshold value can be fix. In some other example, the risk estimator 120 is configured to obtain risk reference data relating to the spatial relationship, such as the distance threshold value. The risk reference data can be received from any data source, such as an ablation planning tool. In some embodiments, the risk estimator is configured to obtain risk reference data for each of the at least one critical region based on at least one of anatomical type of the critical region, subject-specific condition of the critical region, type of the ablation procedure, and user input relating to risk reference data. In an example, the risk reference data can be determined based on a user input, and thus user-configurable. In another example, the risk reference data can be different for different anatomical type of the critical regions. For example, there can be a lookup table defining the risk reference data for each anatomical type, and the risk estimator can be configured to firstly determine the anatomical type of a critical region, and then to obtain the corresponding risk reference by accessing the lookup table. In another example, different risk reference data can be defined for different types of ablation procedure, or for different subject-specific conditions. For example, an unhealthy critical organ can be more vulnerable as compared to a healthy critical organ.

In some embodiments, the risk estimator 120 is configured to obtain thermal distribution of the ablation procedure over the at least one critical region 330, and to obtain risk reference data relating to temperature for each of the at least one critical region 330, and to estimate the impact of the ablation on each of the at least critical region based on the thermal distribution, the risk reference data and the risk alert rule. In case that there are more than one ablation instances during the ablation procedure, the risk estimator 120 can obtain the thermal distribution for each and all ablation instances. The risk estimator 120 can predict the thermal distribution based on the ablation plan (the ablation position, the energy and the duration, etc.), the anatomical positions of tissues, and the heat conductivity or transfer properties of various types of tissues. The anatomical positions and the type of tissues can be, for example, determined by analyzing a medical image. The risk estimator 120 can also measure the actual thermal distribution, such as by means of thermal sensors and/or by means of imaging sensors. The risk estimator 120 can obtain thermal distribution in any suitable way, existing approaches such as those proposed in US US2014/0296842 A1, or any other approaches developed in future.

In some examples, the risk alert rule can be to generate a risk alert for a critical region if determining the temperature of the critical region is above a certain threshold value based on the thermal distribution over the critical region. As already described in the above, the risk alert rule can be to generate more than one types of risk alerts, and each is generated with reference to a different threshold value. In other words, the risk reference data can include more than one temperature threshold values.

In some embodiments, the user interface 110 is configured to visualize the one or more threshold values. In the example illustrated in Fig.3B, the target 320 is ovarian-shaped, and the boundary curve 360 is an isotherm composed by any point at which the temperature is equal to a first threshold value, and the boundary curve 365 is another isotherm composed by any point at which the temperature is equal to a second threshold value. Referring to Fig.3B, the existence of the relatively large vessel 315, as a heat sink, results in the irregular isotherms 360, 365. In some embodiments, the user interface 110 is configure to visualize the thermal distribution (not illustrated in Fig.3B) in the image.

The risk alert information includes one or more risk alert. Each risk alert includes information indicative of the corresponding critical region. For example, each risk alert includes critical region identifier for identifying the corresponding critical region, and the risk alert information can include one or more critical region identifiers. In an embodiment where there are more than one type of risk alert, each risk alert includes a critical region identifier and a risk alert level identifier for identifying the corresponding type of risk alert.

Referring to Fig. 1, the apparatus 100 further comprises a risk alert positioning unit 130 for obtaining alert position information of each of the at least one critical region. The alert position information of a critical region is indicative an alert position for the critical region where the risk alert is to be visualized in the image 300. The alert position being at or adjacent to a position of the critical region in the image 300. The alert position information can be, for example, coordinates or a range of coordinates of the alert position in the image 300. In some embodiments, the alert position for a critical region can be the center of the critical region or has a predetermined position shift relative to the center of the critical region. In some embodiments, the alert position can be determined based on the setting of the image 300 or user preference. The user interface 110 is configured to visualize, in the image, the one or more risk alerts based on the risk alert information and the alert positioning information. Referring to Fig.3A and Fig.3B, the risk alert is visualized as a predetermined icon 340 adjacent to the critical region 330. Indicator other than the predetermined icon 340 can be designed and adopted. In some embodiments, the same icon is used for any critical region because the position of the risk alert indicates to which critical region the risk alert is connected, and there is no need to visualize risk alerts for different critical region as different icons. In some embodiments, different indicators or indicator of different colors can be used for different type of risk alert. Additionally or alternatively, the user interface 110 can visualize the risk alert by applying a special visual effect on the corresponding critical region, such as highlighting, blinking or the like.

The risk estimator 120 is further configured to, in response to any change of the estimated impact, automatically update the risk alert information. The user interface 110 is further configured to, in response to any change of the risk alert information, automatically visualizing, in the image 300, the one or more risk alerts based on the risk alert information and the alert positioning information. Once the risk estimator 120 updates the risk alert information, it will send the updated risk alert information to the user interface 110 without any additional user input, and once the user interface 110 receives any risk alert information, it will update the visualization without any additional user input.

In some embodiments, the risk estimator 120 is further configured to obtain a position change of a critical region 330 relative to the target region 320, and to estimate the impact of the ablation on the critical region 320 based on the position change of the critical region. In an example, the risk estimator 120 is configured to track the position of the critical region, and update the estimated impacts on the at least one critical regions based on the tracked position of the critical region as well as the risk alert information, and in turn the user interface 110 can update the risk alert accordingly. In this way, the risk alert is in real-time and requires no additional manual operation. For example, when the clinicians performance an open surgery to separate the critical region further away from the target region, the proposed apparatus 300 can provide the clinician with real-time risk alert, and the risk alert for a critical region will go off as soon as the critical region is moved out of the risk zone.

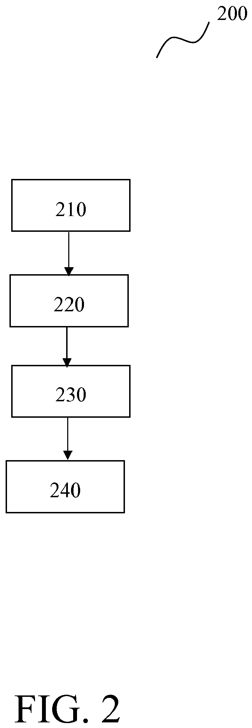

Fig.2 schematically illustrates a method 200 of assisting an ablation procedure on a target region of a subject in accordance with some embodiments of the present invention. The sequence of the steps illustrated in Fig.2 is only schematic and are non-limiting.

In step 220, the impact of the ablation procedure is estimated for each of the at least one critical regions, and risk alert information is generated based on the estimated impacts and a risk alert rule. The risk alert information includes one or more risk alert. In certain cases where it is determined that no risk alert needs to be generated, the risk alert information can be empty or include information indicative of no risk alert. In response to any change of the estimated impact or the risk alert rule, the risk alert information will automatically updated accordingly.

In step 230, alert position information of each of the at least one critical region is obtained. The alert position information of a critical region is indicative an alert position for the critical region in the image where the risk alert is to be visualized, and the alert position being at or adjacent to a position of the critical region in the image. Step 230 can be performed prior to, in parallel or after step 220.

In step 240, the user interface 110 visualizes an image 300 of the target region 320 and at least one critical region 330 of the subject. Upon receiving the risk alert information or any change thereof or any update thereof, the user interface 110 visualizes the one or more risk alerts in the image based on the risk alert information and the alert positioning information.

In some embodiments, in step 210, a medical image is obtained, and the image 300 to be visualized by the user interface 110 is formed based on the medical image. In an embodiment, the at least one critical region are identified by segmenting the medical image.

The technique processes described herein may be implemented by various means. For example, these techniques may be implemented in hardware, software, or a combination thereof. For a hardware implementation, the technical processes may be implemented within one or more application specific integrated circuits (ASICs), digital signal processors (DSPs), digital signal processing devices (DSPDs), programmable logic devices (PLDs), field programmable gate arrays (FPGAs), processors, controllers, microcontrollers, microprocessors, other electronic units designed to perform the functions described herein, or a combination thereof. With software, implementation can be through modules (e.g., procedures, functions, and so on) that perform the functions described herein. The software codes may be stored in a volatile or non-volatile storage medium and executed by the processors.

Moreover, aspects of the claimed subject matter may be implemented as a method, apparatus, system, or article of manufacture using standard programming and/or engineering techniques to produce software, firmware, hardware, or any combination thereof to control a computer or computing components to implement various aspects of the claimed subject matter. The term "article of manufacture" as used herein is intended to encompass a computer program accessible from any computer-readable device, carrier, or media. For example, computer readable media can include but are not limited to magnetic storage devices (e.g., hard disk, floppy disk, magnetic strips...), optical disks (e.g., compact disk (CD), digital versatile disk (DVD)...), smart cards, and flash memory devices (e.g., card, stick, key drive...). Of course, those skilled in the art will recognize that many modifications may be made to this configuration without departing from the scope or spirit of what is described herein.

As used in this application, the terms "risk estimator", "risk alert positioning unit", "processor", are intended to refer to a general-purpose processor, a specific-purpose processor, a computer processor, or a computer-related entity, either hardware, a combination of hardware and software, software, or software in execution. For example, a component may be, but is not limited to, a process running on a processor, a processor, an object, an executable, a thread of execution, a program, and/or a computer. By way of illustration, both an application running on a server and the server can be a component. One or more components may reside within a process and/or thread of execution and a component may be localized on one computer and/or distributed among two or more computers.

What has been described above includes examples of one or more embodiments. It is, of course, not possible to describe every conceivable combination of components or methodologies for the purpose of describing the aforementioned embodiments, but one of ordinary skill in the art may recognize that many further combinations and permutations of various embodiments are possible. Accordingly, the described embodiments are intended to embrace all such alterations, modifications and variations that fall within the spirit and scope of the appended claims. Furthermore, to the extent that the term "includes" is used in either the detailed description or the claims, such term is intended to be inclusive in a manner similar to the term "comprising" as "comprising" is interpreted when employed as a transitional word in a claim.

## Claims

1. An apparatus for assisting an ablation procedure on a target region of a subject, comprising:
a user interface (110) for visualizing an image (300) of the target region (320) and at least one critical region (330) of the subject; and
a risk estimator (120) for estimating an impact of the ablation on each of the at least one critical region, and generating risk alert information based on the estimated impacts and a risk alert rule, the risk alert information including one or more risk alert, each of which including information indicative of the corresponding critical region;
a risk alert positioning unit (130) for obtaining alert position information of each of the at least one critical region, the alert position information of a critical region is indicative an alert position for the critical region in the image where the risk alert is to be visualized, the alert position being at or adjacent to a position of the critical region in the image; wherein
the risk estimator (120) is further configured to, in response to any change of the estimated impact or the risk alert rule, automatically update the risk alert information; and
the user interface (110) is further configured to, in response to any change of the risk alert information, automatically visualizing, in the image (300), the one or more risk alerts (340) based on the risk alert information and the alert positioning information.

2. The apparatus of Claim 1, wherein the risk estimator (120) is further configured to:
obtain spatial relationship between the target region and the at least one critical region;
obtain risk reference data relating to the spatial relationship for each of the at least one critical region; and
estimate the impact of the ablation on each of the at least critical region based on the spatial relationship, the risk reference data and the risk alert rule.

3. The apparatus of Claim 2, wherein the risk reference data includes a distance threshold for a distance relative to the target region, and the user interface (110) is further configured to visualize the distance threshold (350) in the image.

4. The apparatus of Claim 1, wherein the risk estimator (120) is further configured to:
obtain thermal distribution of the ablation procedure over the at least one critical region;
obtain risk reference data relating to temperature for each of the at least one critical region; and
estimate the impact of the ablation on each of the at least critical region based on the thermal distribution, the risk reference data and the risk alert rule.

5. The apparatus of Claim 4, wherein the risk reference data includes a temperature threshold, and the user interface (110) is further configured to visualize the temperature threshold (360, 365) based on the temperature threshold and the temperature distribution.

6. The apparatus of Claim 2 or 4, wherein the risk estimator (120) is configured to obtain the risk reference data for each of the at least one critical region, based on at least one of (i) anatomical type of the critical region, (ii) subject-specific condition of the critical region, (iii) type of the ablation procedure, and (iv) user input relating to risk reference data.

7. The apparatus of Claim 1, wherein the risk estimator (120) is further configured to:
obtain a position change of a critical region relative to the target region; and
update the estimated impact of the ablation on the critical region based on the position change of the critical region.

8. The apparatus of Claim 7, where the risk estimator is further configured to track the position of the critical region.

9. The apparatus of Claim 7, where the risk estimator (120) is further configured to determine the position change of the critical region based on a user input indicative of the position of the critical region or the change of the position of the critical region.

10. The apparatus of Claim 1, wherein the risk estimator (120) is further configured to:
obtain a position of an ablation device; and
estimate the impact of the ablation on each of the at least critical region further based on the obtained position of an ablation device.

11. A method of assisting an ablation procedure on a target region (320) of a subject, comprising steps of:
visualizing (240), via a user interface, an image (300) of the target region (320) and at least one critical region (330) of the subject; and
estimating (220) an impact of the ablation procedure on each of the at least one critical region;
generating risk (220) alert information based on the estimated impacts and a risk alert rule, the risk alert information including one or more risk alert, each of which including information indicative of the corresponding critical region;
obtaining (230) alert position information of each of the at least one critical region, the alert position information of a critical region is indicative an alert position for the critical region in the image where the risk alert is to be visualized, the alert position being at or adjacent to a position of the critical region in the image; wherein
automatically updating (220), in response to any change of the estimated impact or the risk alert rule, the risk alert information; and
automatically visualizing (240), in response to any change of the risk alert information, in the image (300), the one or more risk alerts (340) based on the risk alert information and the alert positioning information.

12. The method of Claim 11, further comprising:
obtaining spatial relationship between the target region and the at least one critical region, and/or thermal distribution of the ablation over the at least one critical region;
obtaining risk reference data for each of the at least one critical region; and
estimating the impact of the ablation on each of the at least critical region based on the risk reference data, the risk alert rule, and the spatial relationship and/or the thermal distribution.

13. The method of Claim 12, wherein the risk reference data for each of the at least one critical region is obtained based on at least one of anatomical type of the critical region, subject-specific condition of the critical region, type of the ablation procedure, and user input relating to risk reference data.

14. The method of Claim 11, further comprising:
obtaining a position change of a critical region relative to the target region; and
estimating the impact of the ablation on the critical region based on the position change of the critical region.

15. A computer-readable medium comprising executable instructions, which when executed, cause a processor to perform any of the steps of claims 11-14.
